# EUROPEAN PATENT SPECIFICATION

(11) **EP 3 510 933 B1**
(45) Date of publication and mention of the grant of the patent: **08.07.2026**
(21) Application number: 18167433.4
(22) Date of filing: 16.04.2018
(51) Int. Cl.: A61B 8/00, A61B 8/08

(54) **ULTRASOUND DIAGNOSIS APPARATUS**
ULTRASCHALLDIAGNOSEVORRICHTUNG
APPAREIL DE DIAGNOSTIC ULTRASONIQUE

(30) Priority: 10.01.2018 KR 20180003351
(43) Date of publication of application: 17.07.2019
(73) Proprietor: Samsung Medison Co., Ltd., Hongcheon-gun, Gangwon-do, 25108 (KR)
(72) Inventor: JANG, Sang-sik, 25108 Gangwon-do (KR); LEE, Jin-yong, 25108 Gangwon-do (KR)
(74) Representative: Arnold & Siedsma

(56) References cited:
- EP-A1- 2 022 404
- WO-A1-2014/039935
- JP-A- 2013 123 582
- US-A1- 2016 038 125
- T. V. VIGNESWARAN ET AL: "Assessment of cardiac angle in fetuses with congenital heart disease at risk of 22q11.2 deletion : Cardiac angle in 22q11.2 deletion", ULTRASOUND IN OBSTETRICS AND GYNECOLOGY, vol. 46, no. 6, 1 December 2015 (2015-12-01), GB, pages 695 - 699, XP055462374, ISSN: 0960-7692, DOI: 10.1002/uog.14832
- COMSTOCK CH: "Normal fetal heart axis and position", OBSTETRICS AND GYNECOLOGY, vol. 70, no. 2, 1 August 1987 (1987-08-01), US, pages 255 - 259, XP055462637, ISSN: 0029-7844
- S. L. BOULTON ET AL: "Cardiac axis in fetuses with abdominal wall defects", ULTRASOUND IN OBSTETRICS AND GYNECOLOGY, vol. 28, no. 6, 1 January 2006 (2006-01-01), GB, pages 785 - 788, XP055462382, ISSN: 0960-7692, DOI: 10.1002/uog.2812
- E. SINKOVSKAYA ET AL: "Defining the fetal cardiac axis between 11 + 0 and 14 + 6 weeks of gestation: experience with 100 consecutive pregnancies", ULTRASOUND IN OBSTETRICS AND GYNECOLOGY, vol. 36, no. 6, 24 November 2010 (2010-11-24), GB, pages 676 - 681, XP055462375, ISSN: 0960-7692, DOI: 10.1002/uog.8814

## Description

### BACKGROUND

### 1. Field

The present disclosure relates to ultrasound diagnosis apparatuses, methods of controlling the ultrasound diagnosis apparatuses, and computer-readable recording media having embodied thereon a program for executing the methods in a computer.

### 2. Description of the Related Art

Ultrasound diagnosis apparatuses transmit ultrasound signals generated by transducers of a probe to an object and detect information about signals reflected from the object, thereby obtaining at least one image of an internal part, for example, soft tissue or blood flow, of the object.

Fetal cardiac anomalies, which are often not detected by blood tests or abnormal examinations, require fetal echocardiography. Using echocardiography, a movement of the heart may be observed in real time, and an anatomical structure of the heart, cardiac function, and intracardiac pressure may be observed in real time and non-invasively.

To determine whether the fetal heart is normal, a method of determining whether an axis of the fetal heart is within a normal range is performed using a four-chamber view of the fetal heart. When determining the axis of the fetal heart, the axis of the heart is determined by using a crux which is a center of a wall of the four-chamber and the spine. In a conventional determination method, a physician manually draws a line through the crux and the spine and calculates an angle between the line and the heart wall. When the calculated angle is near 45 °, the fetal heart is determined to be normal, and when the calculated angle is out of this range, the fetal heart is suspected to be abnormal.

Conventionally, when a physician manually performs such an operation using a tool provided by an ultrasonic apparatus, a read error may occur, and it is difficult to visually detect abnormalities.

T. V. VIGNESWARAN ET AL: "Assessment of cardiac angle in fetuses with congenital heart disease at risk of 22q11.2 deletion", ULTRASOUND IN OBSTETRICS AND GYNECOLOGY, vol. 46, no. 6, 1 December 2015, discloses that the fetal cardiac angle is larger in those with del.22q11.2 compared with those with a normal karyotype and that the fetal cardiac angle was an independent predictor in a logistic regression model.

### SUMMARY OF THE DISCLOSURE

Provided are ultrasound diagnosis apparatuses and methods of controlling the ultrasound diagnosis apparatuses capable of automatically detecting information about an axis of a heart and efficiently diagnosing a heart abnormality through an angle indicator.

Provided are also ultrasound diagnosis apparatuses and methods of controlling the ultrasound diagnosis apparatuses allowing a user to intuitively determine whether a heart axis is abnormal based on information about the heart axis automatically detected and analyzed.

Additional aspects will be set forth in part in the description which follows and, in part, will be apparent from the description, or may be learned by practice of the presented embodiments.

According to an aspect of the invention, an ultrasound diagnosis apparatus according to claim 1 is provided.

According to an aspect which is not within the scope of the claims, a method of controlling an ultrasound diagnosis apparatus includes acquiring an ultrasound image of a two-dimensional (2D) cross-section including a first point representing a crux of a heart of a fetus and a second point representing a center of a spine, based on ultrasound image data of the fetus; displaying a first body axis including a line connecting the first point and the second point on the ultrasound image; displaying a first heart axis corresponding to a heart wall of the heart on the ultrasound image; and displaying an angle indicator indicating angle information about an angle between the first body axis and the first heart axis on the ultrasound image.

According to an aspect which is not within the scope of the claims, a computer-readable recording medium having embodied thereon a program for executing a method of controlling an ultrasound diagnosis apparatus in a computer.

### BRIEF DESCRIPTION OF THE DRAWINGS

These and/or other aspects will become apparent and more readily appreciated from the following description of the embodiments, taken in conjunction with the accompanying drawings in which:
The above and other features and advantages will become more apparent by describing in detail exemplary embodiments thereof with reference to the attached drawings in which reference numerals mean structural elements:
FIG. 1 is a block diagram illustrating an ultrasound diagnosis apparatus according to an exemplary embodiment;
FIGS. 2A, 2B, and 2C are diagrams respectively illustrating an ultrasound diagnosis apparatus;
FIG. 3 is a block diagram showing a configuration of an ultrasound diagnosis apparatus;
FIG. 4 is a flowchart of a method of controlling an ultrasound diagnosis apparatus according to an exemplary embodiment which is not within the scope of the claims;
FIG. 5 shows a schematic diagram of a body and a heart of a fetus included in an ultrasound image acquired by the ultrasound diagnosis apparatus;
FIG. 6A illustrates an ultrasound image displayed by an ultrasound diagnosis apparatus;
FIG. 6B is a diagram for explaining an ultrasound diagnosis apparatus recognizing a body and the heart in an ultrasound image;
FIG. 6C is a diagram for explaining an ultrasound diagnosis apparatus acquiring a first point and a second point in an ultrasound image;
FIG. 6D is a diagram for explaining an ultrasound diagnosis apparatus acquiring a first body axis and a second body axis in an ultrasound image;
FIG. 6E is a diagram for explaining an ultrasound diagnosis apparatus acquiring a heart axis of a fetus in an ultrasound image,;
FIG. 7 illustrates an ultrasound image displayed by an ultrasound diagnosis apparatus together with an angle indicator, according to an exemplary embodiment which is not within the scope of the claims;
FIG. 8 illustrates an ultrasound image displayed by an ultrasound diagnosis apparatus together with an angle indicator, according to the invention;
FIG. 9 illustrates an ultrasound image displayed by an ultrasound diagnosis apparatus, according to an exemplary embodiment which is not within the scope of the claims; and
FIG. 10 is a flowchart of a method of controlling an ultrasound diagnosis apparatus, according to an exemplary embodiment which is not within the scope of the claims.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments may be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

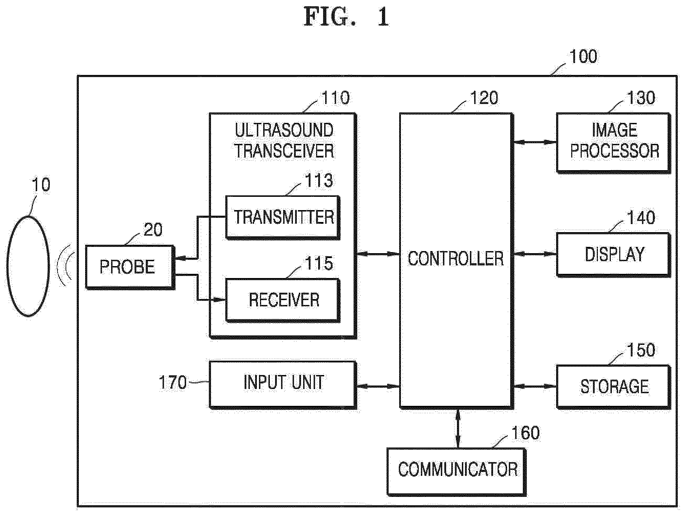

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

In the present embodiment, the probe 20 may include a plurality of transducers. The transducers are arranged in two dimensions (2D), forming a 2D transducer array.

For example, the 2D transducer array may include a plurality of sub-arrays arranged in a first direction, each of the sub-arrays including a plurality of transducers arranged in a second direction that is different from the first direction.

The ultrasound transceiver 110 may include an analog beamformer 113 and a digital beamformer 115. Although FIG. 1 illustrates that the ultrasound transceiver 110 and the probe 20 are provided as being separate from each other, the probe 20 according to the present exemplary embodiment may include the entire ultrasound transceiver 110 or a part of the ultrasound transceiver 110. For example, the probe 20 may include one or both of the analog beamformer 113 and the digital beamformer 115.

The controller 120 may calculate a time delay value for digital beamforming with respect to the sub-arrays included in the 2D transducer array. Also, the controller 120 may calculate a time delay value for analog beamforming for each of the transducers included in any one sub-array of the sub-arrays.

The controller 120 may control the analog beamformer 113 and the digital beamformer 115 to form a transmission signal to be applied to each of the transducers, according to the time delay values for analog beamforming and digital beamforming.

Also, the controller 120 may control the analog beamformer 113 to add signals received from the transducers for each sub-array, according to the time delay value for analog beamforming. Also, the controller 120 may control the ultrasound transceiver 110 to perform analog to digital conversion of the signals added for each sub-array. Also, the controller 120 may control the digital beamformer 115 to generate ultrasound data by adding the digitized signals according to the time delay value for digital beamforming.

The image processor 130 generates an ultrasound image by using generated ultrasound data.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a shortrange communication module, a wired communication module, and a wireless communication module.

The communicator 160 may transmit and receive a control signal and data to and from the external apparatuses.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

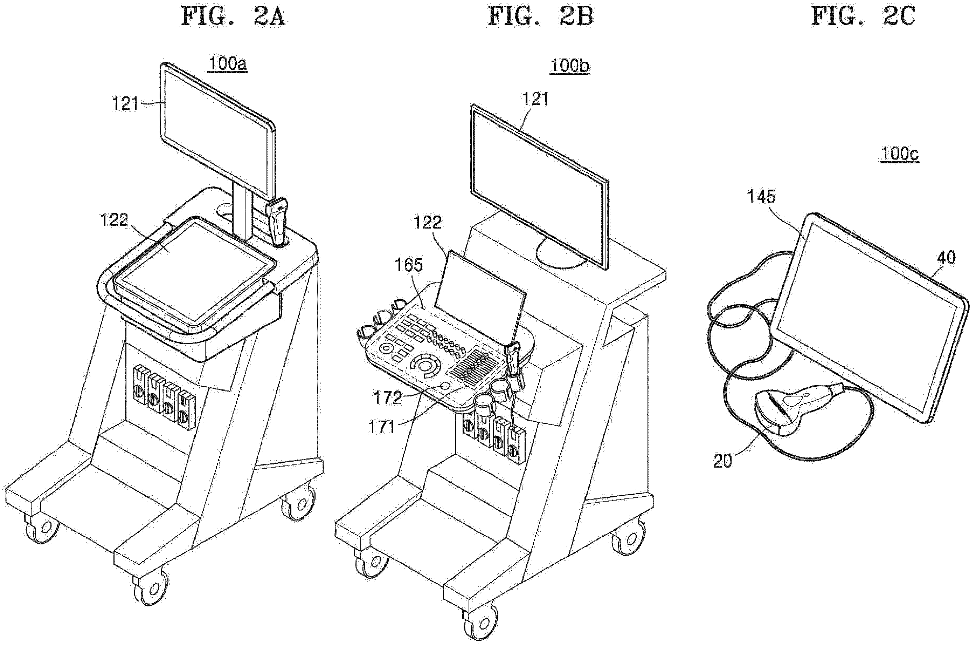

An example of the ultrasound diagnosis apparatus 100 according to the present exemplary embodiment is described below with reference to FIGS. 2A, 2B, and 2C.

FIGS. 2A, 2B, and 2C are diagrams illustrating ultrasound diagnosis apparatus according to an exemplary embodiment.

Referring to FIGS. 2A and 2B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 2B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100 may include a portable device. An example of the portable ultrasound diagnosis apparatus 100 may include, for example, smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

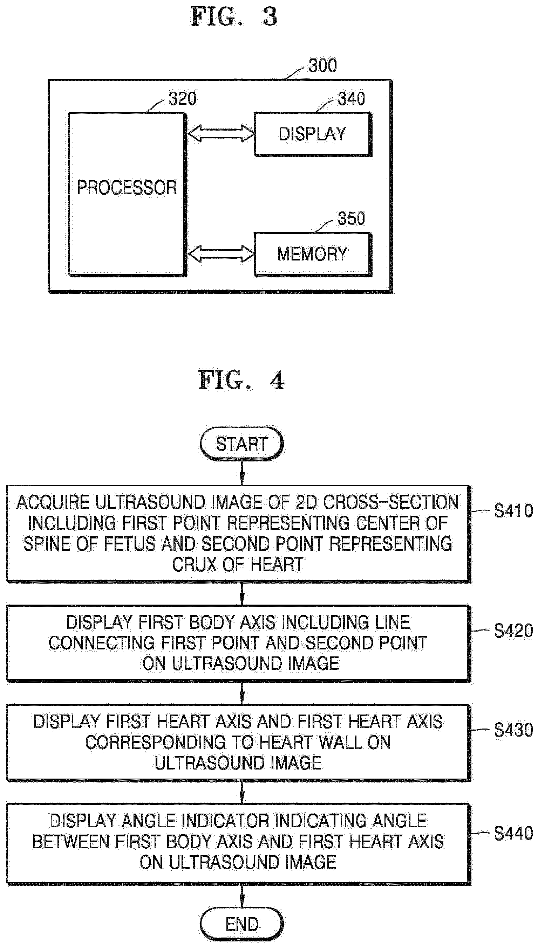

FIG. 3 is a block diagram showing a configuration of the ultrasound diagnosis apparatus 300 according to an exemplary embodiment.

According to an exemplary embodiment, the ultrasound diagnosis apparatus 300 may include all image processing apparatuses capable of acquiring ultrasound images based on ultrasound image data acquired through ultrasound imaging. Also, the ultrasound diagnosis apparatus 300 may include a computing apparatus capable of controlling acquisition of the ultrasound image data through ultrasound imaging.

Also, the ultrasound diagnosis apparatus 300 may be an apparatus having a function of receiving the ultrasound image data acquired through ultrasound imaging, processing and displaying the ultrasound image data. For example, the ultrasound diagnosis apparatus 300 may include a medical server apparatus in a hospital where a patient performs ultrasound imaging or other hospital. The ultrasound diagnosis apparatus 300 is not limited thereto and may include a smart phone, a tablet PC, a PC, a smart TV, a mobile phone, a personal digital assistant (PDA), a laptop, a media player, a digital camera, a home appliance, and other mobile or non-mobile computing apparatuses.

Referring to FIG. 3, the ultrasound diagnosis apparatus 300 may include a processor 320, a display 340, and a memory 350.

The ultrasound diagnosis apparatus 300 may be included in the ultrasound diagnosis apparatus 100 described with reference to FIG. 1. In this case, the display 340 and the memory 350 of the ultrasound diagnosis apparatus 300 may correspond to the display 140 and the storage 150 shown in FIG. 1, respectively. Also, the processor 320 may correspond to one or a combination of the controller 120 and the image processor 130 of FIG. 1.

Also, components of the ultrasound diagnosis apparatus 300 are not limited to the components shown in FIG. 3. According to an exemplary embodiment, the ultrasound diagnosis apparatus 300 may be implemented by more components than the components shown in FIG. 3.

For example, the ultrasound diagnosis apparatus 300 may be implemented integrally with the ultrasound diagnosis apparatus 300 or may further include a detachable probe (not shown) connected to the ultrasound diagnosis apparatus 300 by wired or wirelessly. The probe may acquire ultrasound image data of an object based on a received ultrasound echo signal.

The ultrasound diagnosis apparatus 300 may further include a communicator (not shown) including one or more components that enable communication with at least one of a client apparatus, an external server, and an external database.

The processor 320 according to an exemplary embodiment may generally control the display 340 and the memory 350. The processor 320 may generally control an operation of the ultrasound diagnosis apparatus 300 by executing a program stored in the memory 350. Also, the processor 320 may include one or more processors.

The processor 320 according to an exemplary embodiment acquires an ultrasound image of the object based on the ultrasound image data of the object. For example, the processor 320 may process the acquired ultrasound image data in real time to acquire the ultrasound image. The ultrasound image acquired in real time by the processor 320 may be a still image or a moving image. The processor 320 may acquire the ultrasound image based on ultrasound image data received through an external apparatus or may acquire the ultrasound image by scanning the object with the probe (not shown).

Also, the processor 320 may control the display 340 to display the ultrasound image generated in real time.

The processor 320 according to an exemplary embodiment may acquire an ultrasound image of a two-dimensional (2D) cross-section including a first point representing a center of the spine of a fetus and a second point representing the crux of the heart, based on ultrasound image data of the fetus. The crux of the heart refers to a part where the left ventricle, the right ventricle, the left atrium, and the right atrium are all connected.

Also, the processor 320 displays a first body axis including a line connecting the first point and the second point on the ultrasound image.

Also, the processor 320 controls the display 340 to display a first heart axis corresponding to a heart wall of the heart on the ultrasound image and display an angle indicator indicating angle information between the first body axis and the first heart axis on the ultrasound image.

The angle indicator includes information indicating whether the heart is in a normal state as well as the angle information between the first body axis and the first heart axis. The angle indicator also includes angle range information indicating that the heart is in the normal state.

For example, the angle range information displayed through the angle indicator may indicate that the angle between the first body axis and the first heart axis is within a range from 25° to 65° . In a case where the angle between the first body axis and the first heart axis is within the range from 25° to 65° , the heart of the fetus may be determined to be normal.

Also, the processor 320 may recognize a body of the fetus and acquire an ultrasound image corresponding to a cross-section including the body of the fetus and a four chamber view of the heart. The four chamber view of the heart represents an image acquired to show the left ventricle, the right ventricle, the left atrium, and the right atrium.

Also, the processor 320 may display a second body axis perpendicular to the first body axis on the ultrasound image.

The processor 320 may detect a ventricular septum, which is a boundary between the left ventricle and the right ventricle, and may determine the first heart axis based on a location of the ventricular septum.

Also, the processor 320 displays the angle range information in a different color according to a probability that the heart is in the normal state. Also, the processor 320 displays a color representing the angle between the first body axis and the first heart axis and a color bar representing an angle value corresponding to the color.

Also, the processor 320 may acquire the ultrasound image of the 2D cross-section based on three-dimensional (3D) ultrasound image data of the fetus.

Also, the processor 320 may display a schematic view of the heart of the fetus including position information of the first body axis, position information of the first heart axis, and the angle information and the angle indicator together.

The display 340 according to an exemplary embodiment may display the ultrasound image generated based on the acquired ultrasound image data and information related to the ultrasound image.

FIG. 4 is a flowchart of a method of controlling an ultrasound diagnosis apparatus according to an exemplary embodiment.

The method of controlling the ultrasound diagnosis apparatus according to an exemplary embodiment may be performed, for example, in the ultrasound diagnosis apparatus 300 described above.

In operation S410, the ultrasound diagnosis apparatus 300 may acquire an ultrasound image of a 2D cross-section including a first point representing a center of the spine of a fetus and a second point representing the crux of the heart. The ultrasound image may include at least one of a moving image and a still image. Also, the ultrasound image may include a brightness (B) mode image.

In operation S420, the ultrasound diagnosis apparatus 300 may display a first body axis including a line connecting the first point and the second point on the ultrasound image.

In operation S430, the ultrasound diagnosis apparatus 300 may display the first heart axis and a first heart axis corresponding to a heart wall of the fetus on the ultrasound image.

In operation S440, the ultrasound diagnosis apparatus 300 may display an angle indicator indicating an angle between the first body axis and the first heart axis on the ultrasound image. For example, In a case where the angle between the first body axis and the first heart axis is within a range from 25° to 65° , the heart of the fetus may be determined to have a high probability of being normal.

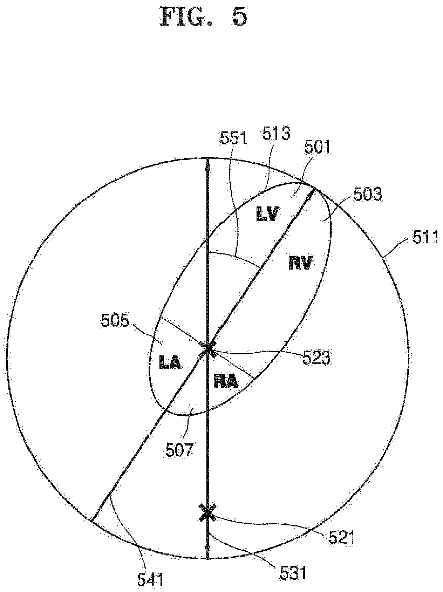

FIG. 5 shows a schematic diagram of a body 511 and a heart 513 of a fetus included in an ultrasound image acquired by the ultrasound diagnosis apparatus 300.

The ultrasound diagnosis apparatus 300 may acquire a first body axis 531 by connecting a first point 521 indicating a center of the spine of the fetus and a second point 523 indicating the crux that is a part where a left ventricle 501, a right ventricle 503, a left ventricle 505 and a right ventricle 507 of the heart 513 of the fetus are all connected.

Also, the ultrasound diagnosis apparatus 300 may acquire a first heart axis 541 based on a position of a ventricular septum, which is a boundary between the left ventricle 501 and the right ventricle 503.

The ultrasound diagnosis apparatus 300 may diagnose whether the heart 513 of the fetus is normal through an angle 551 formed by the first body axis 531 and the first heart axis 541.

Hereinafter, it will be described in detail that the ultrasound diagnosis apparatus 300 acquires and displays the angle 551 between the first body axis 531 and the first heart axis 541 from the ultrasound image.

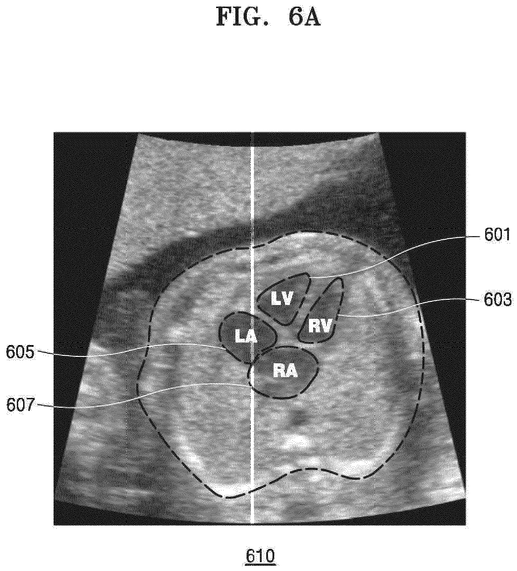

FIG. 6A illustrates an ultrasound image 610 displayed by the ultrasound diagnosis apparatus 300 according to an exemplary embodiment.

The ultrasound diagnosis apparatus 300 may acquire an ultrasound image 610 including a four-chamber view of the heart of a fetus. The four-chamber view of the heart refers to an image acquired to show all a left ventricle 601, a right ventricle 603, a left atrium 605, and a right atrium 607.

The ultrasound image 610 may be, for example, a B mode image. When the ultrasound image 610 is the B mode image, layouts of cross-sections of the left ventricle 601, the right ventricle 603, the left atrium 605, and the right atrium 607 may be well represented in the ultrasound image 610.

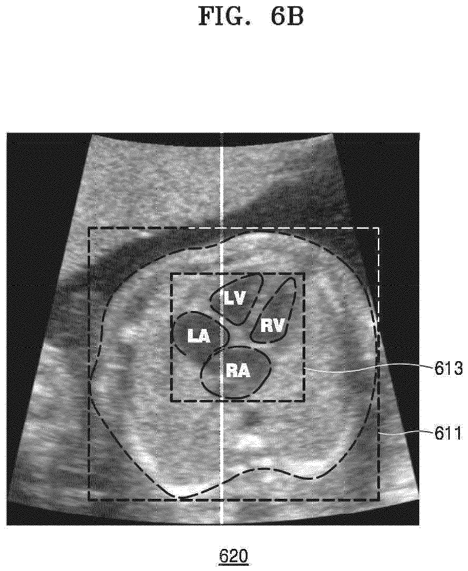

FIG. 6B is a diagram for explaining the ultrasound diagnosis apparatus 300 recognizing a body and the heart in an ultrasound image 620, according to an exemplary embodiment.

The ultrasound diagnosis apparatus 300 may recognize a part 611 including the body of a fetus in the ultrasound image 620. Thereafter, the ultrasound diagnosis apparatus 300 may recognize a heart part 613 including a left ventricle, a right ventricle, a left atrium, and a right atrium within the portion 611 in the part 611 of the body of the fetus.

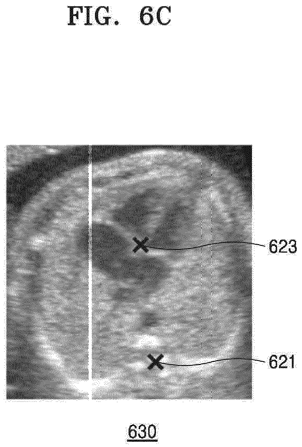

FIG. 6C is a diagram for explaining the ultrasound diagnosis apparatus 300 acquiring a first point 621 and a second point 623 in an ultrasound image 630, according to an exemplary embodiment.

The ultrasound diagnosis apparatus 300 according to an exemplary embodiment may acquire the first point 621 representing a center of the spine of a fetus and the second point 623 representing a crux of the heart in the ultrasound image 630.

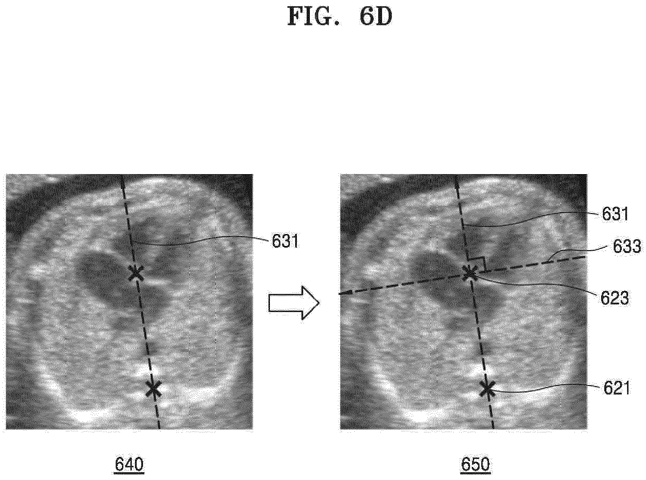

FIG. 6D is a diagram for explaining the ultrasound diagnosis apparatus 300 acquiring a first body axis 631 and a second body axis 633 in an ultrasound image 640, according to an exemplary embodiment.

The ultrasound diagnosis apparatus 300 acquires the first body axis 631 including a line connecting the first point 621 and the second point 623 representing a crux of the heart in the ultrasound image 640 representing a center of the spine of a fetus. The ultrasound diagnosis apparatus 300 displays the first body axis 631 on the ultrasound image 640.

The ultrasound diagnosis apparatus 300 may also acquire the first body axis 631 and the second body axis 633 perpendicular to the first body axis 631. The ultrasound diagnosis apparatus 300 may display the first body axis 631 and the second body axis 633 perpendicular to the first body axis 631 on the ultrasound image 650.

Meanwhile, the ultrasound diagnosis apparatus 300 may acquire the first body axis 631 and the second body axis 631 perpendicular to the first body axis 631 in the ultrasound images 640 and 650 using an existing ultrasound image processing technique.

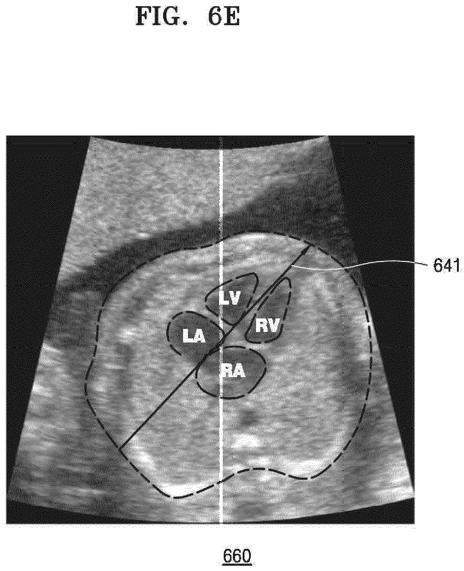

FIG. 6E is a diagram for explaining the ultrasound diagnosis apparatus 300 acquiring a heart axis of a fetus in an ultrasound image 660, according to an exemplary embodiment.

The ultrasound diagnosis apparatus 300 acquires a first heart axis 641 corresponding to a heart wall of the fetus. For example, the ultrasound diagnosis apparatus 300 may acquire the first heart axis 641 based on a location of a ventricular septum, which is a boundary between a left ventricle and a right ventricle at the heart of the fetus. The ultrasound diagnosis apparatus 300 displays the first heart axis 641 on the ultrasound image 660.

The ultrasound diagnosis apparatus 300 may detect the position of the ventricular septum in the ultrasound image 660 using an existing ultrasound image processing technique and acquire the first heart axis 641 corresponding to the ventricular septum.

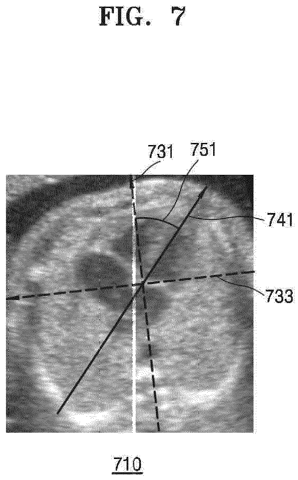

FIG. 7 illustrates an ultrasound image 710 displayed by the ultrasound diagnosis apparatus 300 together with an angle indicator, according to an exemplary embodiment.

The ultrasound diagnosis apparatus 300 may display the angle indicator indicating an angle 751 between the first body axis 731 and the first heart axis 741 on the ultrasound image 710.

The angle indicator may be a user interface displayed on the ultrasound image 710 to indicate the angle 751 formed by the first body axis 731 and the first heart axis 741.

The angle indicator may include various user interfaces for indicating the angle 751 formed by the first body axis 731 and the first heart axis 741. Also, the angle indicator may further include a user interface indicating positions of the first body axis 731 and the first heart axis 741. The angle indicator may further include a user interface indicating a second body axis 733 perpendicular to the first body axis 731.

The angle indicator may further include a value of the angle 751 formed by the first body axis 731 and the first heart axis 741 and may further include a user interface indicating whether the heart of a fetus is normal.

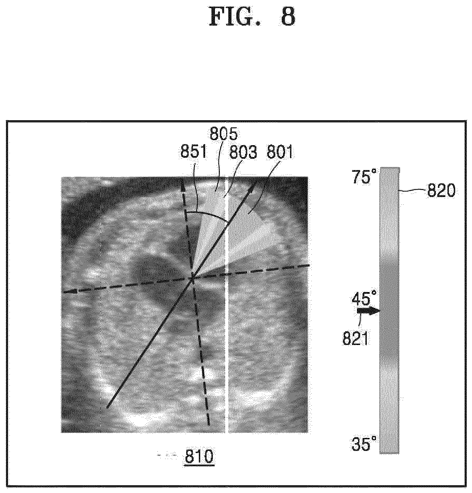

FIG. 8 illustrates an ultrasound image 810 displayed by an ultrasound diagnosis apparatus 300 together with an angle indicator, according to the invention.

The ultrasound diagnosis apparatus 300 may display the angle indicator including a user interface for indicating an angle 851 formed by a first body axis and a first heart axis on the ultrasound image 810.

The angle indicator includes not only angle information between the first body axis and the first heart axis but also information indicating whether the heart is in a normal state. The angle indicator indicates the angle information between the first body axis and the first heart axis and indicates whether the heart is normal based on the angle information.

The angle indicator also includes angle range information 801, 803, and 805 indicating that the heart is in a normal state.

The angle range information 801, 803, and 805 displayed by the ultrasound diagnosis apparatus 300 through the angle indicator may indicate a range of the angle 851 between the first body axis and the first heart axis from 25° to 65°. For example, in a case where the angle 851 between the first body axis and the first heart axis is in the range from 25° to 65°, the heart of a fetus may be determined to be normal.

The ultrasound diagnosis apparatus 300 displays the angle range information 801, 803, and 805 in different colors according to a probability range in which the heart is in the normal state. For example, the ultrasound diagnosis apparatus 300 displays the angle range information 801, 803, and 805 on parts corresponding to between the first body axis and the first heart axis in the ultrasound image 810 in different colors according to the probability range in which the heart is in the normal state.

In FIG. 8, the ultrasound diagnosis apparatus 300 displays the angle range information 801, 803, and 805 on positions corresponding to an angle range in which the heart is in the normal state. However, the ultrasound diagnosis apparatus 300 displays the angle range information 801, 803, and 805 on parts corresponding to an angle range (less than 25° or greater than 65°) in which the heart is abnormal.

The angle range information 801, 803, and 805 may include a user interface for indicating which one of a first range, a second range, and a third range relates to the angle 851 formed by the first body axis and the first heart axis. More specifically, the angle range information 801, 803, and 805 may include first range information 801, second range information 803, and third range information 805. The first range information 801, the second range information 803, and the third range information 805 may be user interfaces corresponding to a probability that the heart is in the normal state.

The first range information 801 may correspond to an angle range having the highest probability that the heart is normal. The second range information 803 may correspond to an angle range having a relatively high probability that the heart is normal. The third range information 805 may correspond to an angle range having a lower probability that the heart is normal than the second range information 803.

For example, the first range information 801 may be a user interface displayed at a part corresponding to the angle 851 between the first body axis and the first heart axis that is the first range from 40° and 50°. The second range information 803 may be a user interface displayed at a part corresponding to the angle the angle 851 between the first body axis and the first heart axis that is the second range from 35° and 55°. The third range information 805 may be a user interface displayed at a part corresponding to the angle the angle 851 between the first body axis and the first heart axis that is the third range from 25° and 65°.

The first range information 801, the second range information 803, and the third range information 805 may be user interfaces displayed in different colors. Also, the first range information 801, the second range information 803, and the third range information 805 may be user interfaces displayed in colors gradually changing according to a change in the corresponding angle.

Also, the ultrasound diagnosis apparatus 300 displays a color bar 820 for displaying an angle corresponding to color values displayed through the angle range information 801, 803, and 805.

The ultrasound diagnosis apparatus 300 displays to a user through the color bar 820 an angle value represented by a color value corresponding to the angle 851 between the first body axis and the first heart axis. For example, the ultrasound diagnosis apparatus 300 may display a point 821 corresponding to the angle 851 between the first body axis and the first heart axis on the color bar 820.

Referring to FIG. 8, the ultrasound diagnosis apparatus 300 may indicate through the first range information 801 that the angle 851 formed by the first body axis and the first heart axis is included in the first range. The ultrasound diagnosis apparatus 300 may display the angle 851 corresponding to a color value displayed through the first range information 801 and the point 821 corresponding to the angle 851.

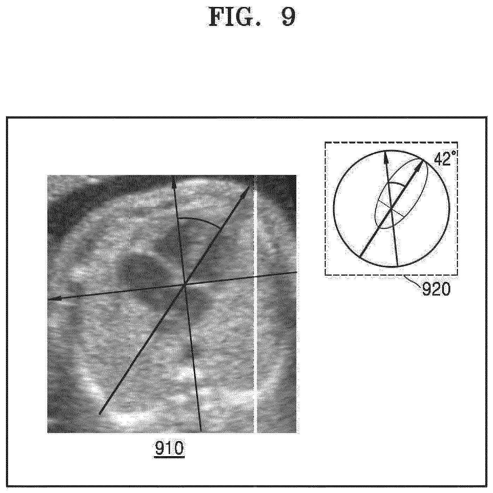

FIG. 9 illustrates an ultrasound image 910 displayed by the ultrasound diagnosis apparatus 300, according to an exemplary embodiment.

The ultrasound diagnosis apparatus 300 according to an exemplary embodiment may display the ultrasound image 910 displaying an angle indicator and a schematic view of a fetal heart 920 including position information of a first body axis, position information of a first heart axis, and angle information simultaneously.

The schematic view 920 of the fetal heart schematically may display a position of the first body axis and a position of the first heart axis of the fetal heart and an angle formed by the first body axis and the first heart axis of the fetal heart.

The ultrasound diagnosis apparatus 300 may overlap and display the schematic view 920 of the fetal heart on a part of the ultrasound image 910 or may not overlap but simultaneously display the ultrasound image 910 and the schematic view 920 of the fetal heart.

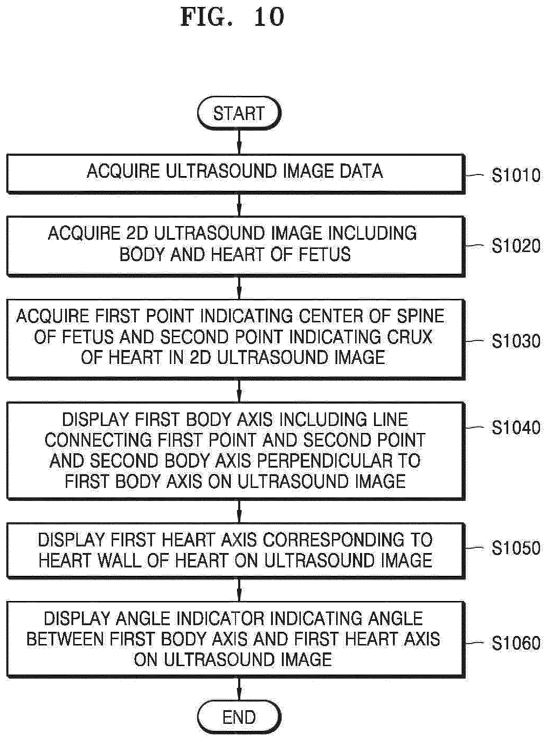

FIG. 10 is a flowchart of a method of controlling an ultrasound diagnosis apparatus, according to an exemplary embodiment.

The method of controlling the ultrasound diagnosis apparatus according to an exemplary embodiment may be performed, for example, in the ultrasound diagnosis apparatus 300 described above.

In operation S1010, the ultrasound diagnosis apparatus 300 may acquire ultrasound image data. The ultrasound image data may be, for example, 3D ultrasound image data.

In operation S1020, the ultrasound diagnosis apparatus 300 may acquire a 2D ultrasound image including a body and the heart of a fetus. Specifically, the ultrasound diagnosis apparatus 300 may acquire the 2D ultrasound image corresponding to a cross-section including the body and the heart of the fetus based on 3D volume data.

In operation S1030, the ultrasound diagnosis apparatus 300 may acquire a first point indicating a center of the spine of the fetus and a second point indicating a crux of the heart in the 2D ultrasound image.

In operation S1040, the ultrasound diagnosis apparatus 300 may display a first body axis including a line connecting the first point and the second point and a second body axis perpendicular to the first body axis on the ultrasound image.

In operation S1050, the ultrasound diagnosis apparatus 300 may display a first heart axis corresponding to a heart wall of the heart on the ultrasound image.

In operation S1060, the ultrasound diagnosis apparatus 300 may display an angle indicator indicating an angle between the first body axis and the first heart axis on the ultrasound image.

Meanwhile, the embodiments may be embodied in the form of a computer-readable recording medium storing instructions executable by a computer and data. The instructions may be stored in the form of program codes, and when executed by a processor, may generate a predetermined program module to perform a predetermined operation. Also, the instructions, when executed by the processor, may perform predetermined operations of the embodiments.

According to the embodiments above, information about an axis of a heart is automatically detected and thus a user may efficiently diagnose a heart abnormality through an angle indicator.

According to the embodiments above, the user may intuitively determine whether a heart axis is abnormal based on information about the heart axis automatically detected and analyzed.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An ultrasound diagnosis apparatus (300) comprising:
a display (340); and
a processor (320) configured to:
acquire an ultrasound image of a two-dimensional cross-section comprising a first point representing a crux of a heart of a fetus and a second point representing a center of a spine, based on ultrasound image data of the fetus,
acquire a first body axis comprising a line connecting the first point and the second point on the ultrasound image, by processing the ultrasound image,
acquire a first heart axis corresponding to a heart wall of the heart on the ultrasound image, by processing the ultrasound image,
control the display (340) to display an angle indicator between the first body axis and the first heart axis on the ultrasound image, and
display, on the ultrasound image, the first body axis, the first heart axis and the angle indicator,
**characterized in that** the angle indicator includes indicating angle information about an angle between the first body axis and the first heart axis and angle range information ((801, 803, 805)) indicating a range of the angle between the first body axis and the first heart axis displayed in different colors according to a probability range in which the heart is in a normal state, the angle indicator thus not only indicating the angle information but also information whether the heart is in the normal state;
and display a color bar (820) for displaying an angle value represented by a color value corresponding to the angle between the first body axis and the first heart axis.

2. The ultrasound diagnosis apparatus (300) of claim 1, wherein the processor (320) is further configured to recognize a body of the fetus and acquire the ultrasound image of the cross-section comprising the body of the fetus and a four-chamber view of the heart.

3. The ultrasound diagnosis apparatus (300) of claim 1, wherein the processor (320) is further configured to display a second body axis perpendicular to the first body axis on the ultrasound image.

4. The ultrasound diagnosis apparatus (300) of claim 1, wherein the processor (320) is further configured to detect a ventricular septum of the heart and determine the first heart axis based on a position of the ventricular septum.

5. The ultrasound diagnosis apparatus (300) of claim 1, wherein the processor (320) is configured to determine that the heart is in the normal state when the angle between the first body axis and the first heart axis is in the angle range.

6. The ultrasound diagnosis apparatus (300) of claim 1, wherein the processor (320) is further configured to acquire the ultrasonic image of the 2D cross-section, based on three-dimensional (3D) ultrasonic image data of the fetus.

7. The ultrasound diagnosis apparatus (300) of claim 1, wherein the processor (320) is further configured to display a schematic view of the heart of the fetus comprising position information of the first body axis, a position of the first heart axis, the angle information, and the angle indicator.

## Patentansprüche

1. Ultraschalldiagnosevorrichtung (300), die Folgendes umfasst:
eine Anzeige (340); und
einen Prozessor (320), der zu Folgendem konfiguriert ist:
Erhalten eines Ultraschallbildes eines zweidimensionalen Querschnitts, der einen ersten Punkt, der ein Crux cordis eines Fötus darstellt, und einen zweiten Punkt, der ein Zentrum einer Wirbelsäule darstellt, umfasst, basierend auf Ultraschallbilddaten des Fötus,
Erhalten einer ersten Körperachse, die eine Linie umfasst, die den ersten Punkt und den zweiten Punkt auf dem Ultraschallbild verbindet, durch Verarbeiten des Ultraschallbildes,
Erhalten einer ersten Herzachse, die einer Herzwand des Herzens auf dem Ultraschallbild entspricht, durch Verarbeiten des Ultraschallbildes,
Steuern der Anzeige (340) zum Anzeigen eines Winkelindikators zwischen der ersten Körperachse und der ersten Herzachse auf dem Ultraschallbild, und
Anzeigen der ersten Körperachse, der ersten Herzachse und des Winkelindikators auf dem Ultraschallbild,
**dadurch gekennzeichnet, dass** der Winkelindikator das Angeben von Winkelinformationen über einen Winkel zwischen der ersten Körperachse und der ersten Herzachse und Winkelbereichsinformationen ((801, 803, 805)) einschließt, die einen Bereich des Winkels zwischen der ersten Körperachse und der ersten Herzachse angeben, der in verschiedenen Farben nach einem Bereich der Wahrscheinlichkeit, dass sich das Herz in einem normalen Zustand befindet, angezeigt wird, wobei der Winkelindikator nicht nur die Winkelinformationen, sondern auch Informationen darüber anzeigt, ob sich das Herz im normalen Zustand befindet;
und Anzeigen eines Farbbalkens (820) zum Anzeigen eines Winkelwertes, der durch einen Farbwert dargestellt wird, der dem Winkel zwischen der ersten Körperachse und der ersten Herzachse entspricht.

2. Ultraschalldiagnosevorrichtung (300) nach Anspruch 1, wobei der Prozessor (320) ferner konfiguriert ist, um einen Körper des Fötus zu erkennen und das Ultraschallbild des Querschnitts zu erhalten, der den Körper des Fötus und eine Vier-Kammer-Ansicht des Herzens umfasst.

3. Ultraschalldiagnosevorrichtung (300) nach Anspruch 1, wobei der Prozessor (320) ferner konfiguriert ist, um auf dem Ultraschallbild eine senkrecht zur ersten Körperachse verlaufende zweite Körperachse anzuzeigen.

4. Ultraschalldiagnosevorrichtung (300) nach Anspruch 1, wobei der Prozessor (320) ferner konfiguriert ist, um ein Ventrikelseptum des Herzens zu erfassen und die erste Herzachse basierend auf einer Position des Ventrikelseptums zu bestimmen.

5. Ultraschalldiagnosevorrichtung (300) nach Anspruch 1, wobei der Prozessor (320) konfiguriert ist, um zu bestimmen, dass sich das Herz im normalen Zustand befindet, wenn der Winkel zwischen der ersten Körperachse und der ersten Herzachse innerhalb des Winkelbereichs liegt.

6. Ultraschalldiagnosevorrichtung (300) nach Anspruch 1, wobei der Prozessor (320) ferner konfiguriert ist, um das Ultraschallbild des 2D-Querschnitts basierend auf dreidimensionalen (3D) Ultraschallbilddaten des Fötus zu erhalten.

7. Ultraschalldiagnosevorrichtung (300) nach Anspruch 1, wobei der Prozessor (320) ferner konfiguriert ist, um eine schematische Ansicht des Herzens des Fötus anzuzeigen, die Positionsinformationen der ersten Körperachse, eine Position der ersten Herzachse, die Winkelinformationen und den Winkelindikator umfasst.

## Revendications

1. Appareil d'échographie diagnostique (300) comprenant :
un écran (340), et
un processeur (320) conçu pour :
acquérir une image échographique d'une coupe transversale bidimensionnelle comprenant un premier point représentant la crux cordis d'un fœtus et un deuxième point représentant le centre de la colonne vertébrale, à partir de données d'image échographique du fœtus,
acquérir un premier axe corporel comprenant une ligne reliant le premier point et le deuxième point sur l'image échographique, par traitement de l'image échographique,
acquérir un premier axe cardiaque correspondant à une paroi cardiaque sur l'image échographique, par traitement de l'image échographique,
commander à l'écran (340) d'afficher un indicateur d'angle entre le premier axe corporel et le premier axe cardiaque sur l'image échographique, et
afficher, sur l'image échographique, le premier axe corporel, le premier axe cardiaque et l'indicateur d'angle ;
**caractérisé en ce que** l'indicateur d'angle comprend l'indication d'informations portant sur l'angle entre le premier axe corporel et le premier axe cardiaque et d'informations portant sur la plage d'angle ((801, 803, 805)) indiquant une plage de l'angle entre le premier axe corporel et le premier axe cardiaque affichée en différentes couleurs selon une plage de probabilité selon laquelle le cœur se trouve dans un état normal, l'indicateur d'angle n'indiquant ainsi pas seulement des informations portant sur l'angle mais aussi des informations portant sur le fait que le cœur se trouve ou non dans un état normal ;
et afficher une barre de couleur (820) pour afficher une valeur d'angle représentée par une valeur de couleur correspondant à l'angle existant entre le premier axe corporel et le premier axe cardiaque.

2. Appareil d'échographie diagnostique (300) selon la revendication 1, dans lequel le processeur (320) est en outre conçu pour reconnaître le corps du fœtus et acquérir l'image échographique de la section transversale comprenant le corps du fœtus et une vue à quatre cavités du cœur.

3. Appareil d'échographie diagnostique (300) selon la revendication 1, dans lequel le processeur (320) est en outre conçu pour afficher un deuxième axe corporel perpendiculaire au premier axe corporel sur l'image échographique.

4. Appareil d'échographie diagnostique (300) selon la revendication 1, dans lequel le processeur (320) est en outre conçu pour détecter **un** septum ventriculaire cardiaque et déterminer le premier axe cardiaque en fonction de la position du septum ventriculaire.

5. Appareil d'échographie diagnostique (300) selon la revendication 1, dans lequel le processeur (320) est conçu pour déterminer que le cœur se trouve dans un état normal lorsque l'angle entre le premier axe corporel et le premier axe cardiaque se trouve dans la plage d'angle.

6. Appareil d'échographie diagnostique (300) selon la revendication 1, dans lequel le processeur (320) est en outre conçu pour acquérir l'image échographique de la section transversale 2D compte tenu de données d'image échographique tridimensionnelle (3D) du fœtus.

7. Appareil d'échographie diagnostique (300) selon la revendication 1, dans lequel le processeur (320) est en outre conçu pour afficher une vue schématique du cœur du fœtus comprenant des informations de position du premier axe corporel, la position du premier axe cardiaque, les informations d'angle et l'indicateur d'angle.
